# EUROPÄISCHE PATENTSCHRIFT

(11) **EP 0 772 491 B1**
(45) Veröffentlichungstag und Bekanntmachung des Hinweises auf die Patenterteilung: **22.09.1999**
(21) Anmeldenummer: 95925846.8
(22) Anmeldetag: 07.07.1995
(51) Int. Cl.: B01J 29/04, C07D 301/12

(54) **OXIDATIONSKATALYSATOR, UND OXIDATIONSVERFAHREN UNTER VERWENDUNG DES OXIDATIONSKATALYSATORS**
OXIDATION CATALYST AND OXIDATION PROCESS USING SAID OXIDATION CATALYST
CATALYSEUR D'OXYDATION ET PROCEDE D'OXYDATION UTILISANT CE CATALYSEUR

(30) Priorität: 20.07.1994 DE 4425672
(43) Veröffentlichungstag der Anmeldung: 14.05.1997
(73) Patentinhaber: BASF AKTIENGESELLSCHAFT, 67056 Ludwigshafen (DE)
(72) Erfinder: MÜLLER, Ulrich, D-67434 Neustadt (DE); LINGELBACH, Peter, D-67063 Ludwigshafen (DE); BASSLER, Peter, D-68519 Viernheim (DE); HARDER, Wolfgang, D-69469 Weinheim (DE); ELLER, Karsten, D-67059 Ludwigshafen (DE); KOHL, Veronika, D-64295 Darmstadt (DE); DEMBOWSKI, Jürgen, D-67307 Göllheim (DE); RIEBER, Norbert, D-68259 Mannheim (DE); FISCHER, Martin, D-67071 Ludwigshafen (DE)
(86) Internationale Anmeldenummer: EP9502651
(87) Internationale Veröffentlichungsnummer: WO9602323

(56) Entgegenhaltungen:
- EP-A- 0 190 609
- EP-A- 0 230 949
- EP-A- 0 325 053
- EP-A- 0 326 759
- EP-A- 0 469 662
- WO-A-87/02910

## Beschreibung

Die vorliegende Erfindung betrifft einen neuen Oxidationskatalysator auf Basis von Titan- oder Vanadiumsilikaliten mit Zeolith-Struktur und einem Gehalt an Platinmetallen sowie verschiedene Oxidationsverfahren unter Verwendung dieses Oxidationskatalysators.

Platinmetallhaltige Titansilikalite sind als Oxidationskatalysatoren bekannt. So wird in der Literaturstelle J.Chem. Soc., Chem.Commun., 1992, S. 1446-1447 (1) die Hydroxylierung von Benzol und Hexan über palladiumhaltigen Titansilikaliten beschrieben. Die JP-OS 92/352771 (2) betrifft die Herstellung von Propylenoxid aus Propen, Wasserstoff und Sauerstoff unter Verwendung eines palladiumhaltigen Titansilikalit-Katalysators. Die EP-A 469 662 (3) offenbart die Eignung edelmetallhaltiger Titansilikalit-Katalysatoren für die Oxidation paraffinischer Verbindungen zu den entsprechenden Alkohol- und/oder Keton-Derivaten.

Derartige aus dem Stand der Technik bekannte Oxidationskatalysatoren weisen jedoch Nachteile auf. Vielfach sind die Katalysatoren nur für einen eng begrenzten Anwendungszweck geeignet. Selektivität, Umsatz, Raum-Zeit-Ausbeute oder Lebensdauer sind auch noch oft verbesserungsbedürftige Parameter.

Aufgabe der vorliegenden Erfindung war es, einen universell verwendbaren, einfach herzustellenden und effizient wirkenden Oxidationskatalysator bereitzustellen, der die Nachteile des Standes der Technik nicht mehr aufweist.

Demgemäß wurde ein Oxidationskatalysator auf Basis von Titan- oder Vanadiumsilikaliten mit Zeolith-Struktur mit einem Gehalt von 0,01 bis 20 Gew.-% an einem oder mehreren Platinmetallen aus der Gruppe Ruthenium, Rhodium, Palladium, Osmium, Iridium und Platin, wobei die Platinmetalle jeweils in mindestens zwei verschiedenen Bindungsenergiezuständen vorliegen und durch Imprägnieren des Oxidationskatalysators mit Salzlösungen der Platinmetalle in der Oxidationsstufe +2 bis +4 und anschließende Hydrierung des getrockneten Katalysators in einer Wasserstoffatmosphäre bei Temperaturen von 20 bis 120° derart aufgebracht sind, daß keine Metall-Metall-Bindungen wirksam werden, gefunden, welcher dadurch gekennzeichnet ist, daß die Titan- oder Vanadiumsilikalite eine Pentasil-Zeolith-Struktur mit röntgenographischer Zuordnung zur MFI-, MEL- oder MFI/MEL-Mischstruktur aufweisen.

Im Sinne der Erfindung ist es von Bedeutung, daß der Oxidationskatalysator vor seinem Einsatz die Platinmetalle in der genannten speziellen Modifikation der Mischung aus verschiedenen Bindungsenergiezuständen enthält. Die verschiedenen Bindungsenergiezustände entsprechen formell verschiedenen Oxidationsstufen der Metalle. In einer bevorzugten Ausführungsform liegen zwei, drei, vier oder fünf verschiedene Bindungsenergiezustände vor.

Beim Vorliegen von zwei verschiedenen Bindungsenergiezuständen kann dies beispielsweise eine Mischung aus Species der Oxidationsstufe 0 und +1, 0 und +2, 0 und +3 oder 0 und +4 sein. Die beiden Species liegen normalerweise im Verhältnis von 5:95 bis 95:5, insbesondere 10:90 bis 90:10 vor.

Beim Vorliegen von drei verschiedenen Bindungsenergiezuständen kann dies beispielsweise eine Mischung aus Species der Oxidationsstufe 0, +1 und +2 oder 0, +2 und +3 oder 0, +2 und +4 oder 0, +1 und +3 oder 0, +1 und +4 oder 0, +3 und +4 sein. Die drei Species liegen normalerweise im Verhältnis von (0,05-20):(0,05-20):1, insbesondere (0,1-10):(0,1-10):1 vor.

Es können weiterhin auch Mischungen aus vier oder mehr verschiedenen Oxidationsstufen vorliegen, beispielsweise aus 0, +1, +2 und +3 oder 0, +1, +2 und +4 oder 0, +2, +3 und +4 oder 0, +1, +3 und +4 oder 0, +1, +2, +3 und +4. Die Species liegen hierbei in ähnlichen Gewichtsverhältnissen zueinander wie bei den Mischungen aus 2 oder 3 verschiedenen Oxidationsstufen vor.

Unter den Platinmetallen wird Palladium bevorzugt. In einer besonders bevorzugten Ausführungsform liegt das Palladium in zwei oder drei verschiedenen Bindungsenergiezuständen vor.

Die Bindungsenergiezustände an der Oberfläche des Katalysators können am einfachsten durch Röntgenphotoelektronenspektroskopie (XPS) charakterisiert werden. So liegen bei einer typischen Mischung von drei Palladiumspecies die entsprechenden Werte für die Energien des Pd-3d_{5/2}-Zustandes bei 335,0-335,4 eV, 336-336,6 eV und 337,1-337,9 eV, was formell den Oxidationsstufen Pd°, Pd¹⁺ und Pd²⁺ entspricht.

Bei den erfindungsgemäßen Oxidationskatalysatoren ist es notwendig, die Platinmetalle derart aufzubringen, daß keine Metall-Metall-Bindungen wirksam werden und Metall-Zeolith-Bindungen überwiegen. Insbesondere aus Untersuchungen der Röntgenfeinstruktur (EXAFS) geht hervor, daß es beim Vorliegen von Palladium wesentlich ist, daß nahezu ausschließlich Palladium-Sauerstoff-Bindungsabstände von 2,02 + 0,02 Å auftreten und Palladium-Palladium-Abstände wie bei ausgedehntem Palladium-Metall oder Palladium-Agglomeraten von 2,74 + 0,02 Å sowie Palladium-Palladium-Abstände von 3,04 + 0,02 Å wie in Palladium(II)-oxid vermieden werden.

Die Basis für den erfindungsgemäßen Oxidationskatalysator bilden bekannte Titan- oder Vanadiumsilikalite mit Pentasil-Zeolith-Struktur mit röntgenographischer Zuordnung zur MFI-, MEL- oder MFI/MEL-Mischstruktur. Zeolithe dieses Typs sind beispielsweise in W.M. Meier und D.H. Olson, "Atlas of Zeolite Structure Types", Butterworths, 2nd Ed., 1987, beschrieben.

Im erfindungsgemäßen Oxidationskatalysator kann das Titan des Silikalits teilweise oder vollständig durch Vanadium ersetzt sein. Das molare Verhältnis von Titan und/oder Vanadium zur Summe aus Silicium plus Titan und/oder Vanadium liegt in der Regel im Bereich von 0,01:1 bis 0,1:1.

Der Gehalt an den genannten Platinmetallen im erfindungsgemäßen Oxidationskatalysator beträgt 0,01 bis 20 Gew.-%, vorzugsweise 0,1 bis 10 Gew.-%, insbesondere 0,2 bis 5 Gew.-%, bezogen auf die Gesamtmasse des Oxidationskatalysators.

Außer mit den genannten Platinmetallen kann der erfindungsgemäße Oxidationskatalysator noch zusätzlich mit einem oder mehreren Elementen aus der Gruppe Eisen, Kobalt, Nickel, Rhenium, Silber und Gold modifiziert sein. Diese Elemente sind dann üblicherweise in einer Menge von 0,01 bis 10 Gew.-%, insbesondere 0,05 bis 5 Gew.-%, bezogen auf die Gesamtmasse des Oxidationskatalysators, enthalten.

Der erfindungsgemäße Oxidationskatalysator wird durch Imprägnieren von Titan- oder Vanadiumsilikaliten mit Zeolith-Struktur mit Salzlösungen der Platinmetalle hergestellt, wobei man im Anschluß an die Imprägnierung durch geeignete reduzierende Bedingungen die erforderliche Verteilung der Bindungsenergiezustände der Platinmetalle einstellt.

So erfolgt das Aufbringen der Platinmetalle durch Imprägnieren mit einer Platinmetallsalzlösung in der Oxidationsstufe +2 bis +4, beispielsweise aus rein wäßriger, rein alkoholischer oder wäßrig-alkoholischer Mischung bei Temperaturen von 20 bis 90°C, insbesondere 30 bis 55°C. Als Salze können dabei z.B. die entsprechenden Chloride, Acetate oder deren Tetramin-Komplexe eingesetzt werden, im Fall von Palladium sollen hier Palladium(II)-chlorid, Palladium(II)-acetat und der Palladium(II)-tetraminchloro-Komplex genannt werden. Hierbei ist die Menge der Metallsalze so zu wählen, daß auf dem resultierenden Oxidationskatalysator Konzentrationen von 0,01 bis 20 Gew.-% an Platinmetall erzielt werden.

Nach gegebenenfalls erforderlicher Trocknung und/oder gegebenenfalls einem Brennschritt der so erhaltenen Katalysatorvorstufe wird die Verteilung der Bindungsenergiezustände durch partielle Reduktion vorliegender höherer Oxidationsstufen der Platinmetalle durch Hydrierung in einer Wasserstoffatmosphäre eingestellt.

Im Sinne der vorliegenden Erfindung wird der erfindungsgemäße Oxidationskatalysator mit Salzlösungen der Platinmetalle in der Oxidationsstufe +2 bis +4 imprägniert und anschließend der getrocknete Katalysator in einer Wasserstoffatmosphäre bei Temperaturen von 20 bis 120°C, insbesondere 25 bis 100°C, vor allem 30 bis 70°C, hydriert.

Wird bei dieser partiellen Reduktion durch Hydrierung in einer Wasserstoffatmosphäre die Temperatur zu hoch gewählt, liegen die Platinmetalle nahezu ausschließlich in der Oxidationsstufe 0, d.h. als Metalle, und in Form größerer Agglomerate vor, was im mikroskopischem Bild am Auftreten von Metall-Clustern mit Größen über 1,0 nm erkennbar ist.

Die vorgenannten Titan- oder Vanadiumsilikalite mit Zeolith-Struktur, insbesondere hierbei solche mit MFI-Pentasil-Zeolith-Struktur, werden in der Regel hergestellt, indem man eine Synthesegel, bestehend aus Wasser, einer Titan- bzw. Vanadiumquelle und Siliciumdioxid in geeigneter Weise unter Zusatz von organischen stickstoffhaltigen Verbindungen ("Schablonen-Verbindungen") unter hydrothermalen Bedingungen und gegebenenfalls unter Zusatz von Ammoniak, Alkali oder Fluorid als Mineralisatoren kristallisiert. Als organische stickstoffhaltige Verbindungen kommen beispielsweise 1,6-Diaminohexan oder Salze oder das freie Hydroxid von Tetraalkylammonium, speziell von Tetrapropylammonium, in Betracht.

Bei der Herstellung der Titan- bzw. Vanadiumsilikalite muß eine Verunreinigung mit größeren Mengen an Alkali- oder Erdalkalimetallverbindungen vermieden werden; Alkaligehalte (insbesondere an Natrium oder Kalium) <100 ppm sind erstrebenswert, um später einen ausreichend aktiven Oxidationskatalysator zu erhalten.

Die Kristallisation der phasenreinen Struktur des Titan- bzw. Vanadiumsilikalits erfolgt vorzugsweise bei Temperaturen von 140-190°C, insbesondere 160-180°C, innerhalb einer Zeitdauer von 2 bis 7 Tagen, wobei bereits nach ca. 4 Tagen gut kristallines Produkt erhalten wird. Durch starkes Rühren und einen hohen pH-Wert von 12-14 während der Kristallisation kann die Synthesedauer einerseits und die Kristallitgröße andererseits deutlich verringert werden.

Von Vorteil sind beispielsweise Primärkristallite von 0,05 bis 0,5 µm, insbesondere aber solche mit Größen von weniger als 0,2 µm im mittleren Partikeldurchmesser.

Nach der Kristallisation kann der Titan- bzw. Vanadiumsilikalit nach an sich bekannten Methoden abfiltriert, gewaschen und bei 100-120°C getrocknet werden.

Zur Entfernung der in den Poren noch vorliegenden Amin- oder Tetraalkylammoniumverbindungen kann das Material noch einer thermischen Behandlung an Luft oder unter Stickstoff unterzogen werden. Dabei ist es vorteilhaft, das Abbrennen des Templates unter Bedingungen vorzunehmen, die den Temperaturanstieg auf Werte <550°C begrenzen.

Zur Modifizierung des erfindungsgemäßen Oxidationskatalysators können außer den schon genannten Zusätzen von Platinmetallen und sonstigen Elementen die nach dem derzeitigen Stand der Technik bekannten Methoden der Verformung unter Zuhilfenahme eines Binders, des Ionenaustausches und der Oberflächenmodifizierung, beispielsweise über chemical vapor deposition (CVD) oder chemische Derivatisierung wie etwa Silylierung, zum Einsatz gelangen.

Das Vorliegen der für eine Oxidationsreaktion benötigen Katalysatorfunktionen kann durch IR-Spektroskopie geprüft werden: bei 550 cm⁻¹ und bei 960 cm⁻¹ treten signifikante Banden auf, die das Vorliegen der erwünschten Festkörper-Kristallinität sowie der benötigten Oxidationsaktivität anzeigen.

Der erfindungsgemäße Oxidationskatalysator kann bei einer Reihe vor Oxidationsreaktionen mit guter Wirkung eingesetzt werden. Von besonderem Interesse sind hier die Epoxidierung von Olefinen und die Herstellung von Wasserstoffperoxid.

Gegenstand der vorliegenden Erfindung ist daher auch ein Verfahren zur Herstellung von Epoxiden aus Olefinen, Wasserstoff und Sauerstoff, welches dadurch gekennzeichnet ist, daß man die Olefine heterogenkatalytisch unter Verwendung des erfindungsgemäßen Oxidationskatalysators umsetzt.

Abhängig vom umzusetzenden Olefin kann die erfindungsgemäße Epoxidierung in flüssiger Phase, in der Gasphase oder auch in überkritischer Phase durchgeführt werden. Dabei wird der Katalysator bei Flüssigkeiten vorzugsweise als Suspension eingesetzt, während bei Gasphasen- oder überkritischer Fahrweise eine Festbettanordnung von Vorteil ist.

Wird die Epoxidierung in flüssiger Phase vorgenommen, arbeitet man vorteilhafterweise bei einem Druck von 1 bis 10 bar und in einer Suspensionsfahrweise in Gegenwart von Lösungsmitteln. Als Lösungsmittel eignen sich Alkohole, z.B. Methanol, Ethanol, iso-Propanol oder tert.-Butanol oder Mischungen hieraus und insbesondere Wasser. Man kann auch Mischungen der genannten Alkohole mit Wasser einsetzen. In bestimmten Fällen bewirkt die Verwendung von Wasser oder wasserhaltigen Lösungsmittelsystemen eine deutliche Selektivitätssteigerung des gewünschten Epoxids gegenüber den reinen Alkoholen als Lösungsmittel.

Die erfindungsgemäße Epoxidierung wird in der Regel bei Temperaturen von -5 bis 70°C, insbesondere 20 bis 50°C, vorgenommen. Das Molverhältnis von Wasserstoff zu Sauerstoff kann üblicherweise im Bereich H₂:O₂ = 1:10 bis 1:1 variiert werden und ist besonders günstig bei 1:2,5 bis 1:1. Das molare Verhältnis von Sauerstoff zu Olefin liegt in der Regel bei 1:1 bis 1:3, vorzugsweise 1:1,5 bis 1:1,7. Als Trägergas kann ein beliebiges Inertgas zugefahren werden, insbesondere eignet sich Stickstoff.

Das eingesetzte Olefin kann eine beliebige organische Verbindung sein, die mindestens eine ethylenisch ungesättigte Doppelbindung enthält. Sie kann aliphatischer, aromatischer oder cycloaliphatischer Natur sein, sie kann aus einer linearen oder einer verzweigten Struktur bestehen. Vorzugsweise enthält das Olefin 2 bis 30 C-Atome. Mehr als eine ethylenisch ungesättigte Doppelbindung kann vorhanden sein, so etwa in Dienen oder Trienen. Das Olefin kann zusätzlich funktionelle Gruppen wie Halogenatome, Carboxylgruppen, Carbonesterfunktionen, Hydroxylgruppen, Etherbrücken, Sulfidbrücken, Carbonylfunktionen, Cyanogruppen, Nitrogruppen oder Aminogruppen enthalten.

Typische Beispiele für derartige Olefine sind Ethylen, Propen, 1-Buten, cis- und trans-2-Buten, 1,3-Butadien, Pentene, Isopren, Hexene, Octene, Nonene, Decene, Undecene, Dodecene, Cyclopenten, Cyclohexen, Dicyclopentadien, Methylencyclopropan, Vinylcyclohexan, Vinylcyclohexen, Allylchlorid, Acrylsäure, Methacrylsäure, Crotonsäure, Vinylessigsäure, Allylalkohol, Alkylacrylate, Alkylmethacrylate, Ölsäure, Linolsäure, Linolensäure, Ester und Glyceride derartiger ungesättigter Fettsäuren, Styrol, α-Methylstyrol, Divinylbenzol, Inden und Stilben. Auch Mischungen der genannten Olefine können nach dem erfindungsgemäßen Verfahren epoxidiert werden.

Das erfindungsgemäße Verfahren eignet sich in besonderem Maße für die Epoxidierung von Propen zu Propylenoxid.

Gegenstand der vorliegenden Erfindung ist auch ein Verfahren zur Herstellung von Wasserstoffperoxid aus Wasserstoff und Sauerstoff, welches dadurch gekennzeichnet ist, daß man die Umsetzung heterogenkatalytisch unter Verwendung des erfindungsgemäßen Oxidationskatalysators durchführt.

Wie bei der erfindungsgemäßen Epoxidierung kann man auch hier in flüssiger Phase in Suspensionsfahrweise oder in der Gasphase oder in überkritischer Phase mit einer Festbettanordnung arbeiten. Bezüglich der Temperatur und mitzuverwendender Lösungsmittel gilt ebenfalls das dort Gesagte. Der Druck kann in einem trägergashaltigen System bis zu 100 bar betragen. Das molare Verhältnis von H₂:O₂ liegt üblicherweise bei 1:15 bis 1:1, insbesondere 1:10 bis 1:1.

Eine Regenerierung des erfindungsgemäßen Oxidationskatalysators ist ebenfalls in einfacher Weise möglich. Desaktivierte Katalysatoren können durch kontrolliertes Abbrennen von Kohlenstoffbelegungen im Temperaturbereich von 350 bis 650°C und nachfolgende Reduktion mit beispielsweise Wasserstoff wieder in eine aktive Form zurückgeführt werden.

Bei geringer Belegung kann der Katalysator auch durch einen einfachen Waschprozeß wieder regeneriert werden. Je nach Bedarf kann der Waschvorgang im neutralen, sauren oder basischen pH-Bereich durchgeführt werden. Gegebenenfalls kann auch mittels einer mineralsauren Wasserstoffperoxidlösung die Katalysatoraktivität wieder regeneriert werden.

Die nachfolgenden Beispiele sollen die Erfindung näher beschreiben, ohne daß jedoch dadurch eine Einschränkung zu verstehen wäre.

### Beispiel 1

Dieses Beispiel beschreibt die Kristallisation eines Titansilikalits.

Dazu wurden in einem Vierhalskolben (2 l Inhalt) 455 g Tetraethylorthosilikat vorgelegt und aus einem Tropftrichter innerhalb von 30 min mit 15 g Tetraisopropylorthotitanat unter Rühren (250 U/min, Blattrührer) versetzt. Es bildete sich eine farblose, klare Mischung. Abschließend versetzte man mit 800 g einer 20 gew.-%igen wäßrigen Tetrapropylammoniumhydroxid-Lösung (Alkaligehalt < 10 ppm) und rührte noch eine Stunde nach. Bei 90°C bis 100°C wurde das durch Hydrolyse gebildete Alkoholgemisch (ca. 450 g) abdestilliert. Man füllte mit 1,5 l deionisiertem Wasser auf und gab das mittlerweise leicht opaque Sol in einen 2,5 l-fassenden Rührautoklaven. Mit einer Heizrate von 3°C/min wurde der verschlossene Autoklav (Ankerrührer, 200 U/min) auf eine Reaktionstemperatur von 175°C gebracht. Nach 92 Stunden wurde die Reaktion beendet. Das erkaltete Reaktionsgemisch (weiße Suspension) wurde abzentrifugiert und mehrfach mit Wasser neutralgewaschen. Der erhaltene Feststoff wurde bei 110°C innerhalb von 24 Stunden getrocknet (Auswaage 149 g). Abschließend wurde unter Luft bei 500°C in 5 Stunden das im Zeolithen noch vorhandene Templat abgebrannt (Kalzinierungsverlust: 14 Gew.-%).

Das reinweiße Produkt hatte nach naßchemischer Analyse einen Ti-Gehalt von 1,5 Gew.-% und einen Restgehalt an Alkali (Kalium) unterhalb von <0,01 Gew.-%. Die Ausbeute (auf eingesetztes SiO₂ gerechnet) betrug 97 %. Die Kristallitgröße lag bei ca. 0,1 - 0,15 µm und das Produkt zeigte im IR typische Banden bei 960 cm⁻¹ und 550 cm⁻¹.

### Beispiel 2

Zur Imprägnierung mit Palladium wurde zunächst mit 0,515 g Palladium(II)-chlorid und 120 g Ammoniaklösung (25 Gew.-% in Wasser) unter Rühren bei Raumtemperatur eine fleischfarbene Lösung hergestellt. In einem Rundkolben wurden 60 g des frisch hergestellten Titansilikalits aus Beispiel 1 in 130 g deionisiertem Wasser suspendiert. Dazu gab man die Gesamtmenge der vorbereiteten Pd-Tetraminchloro-Komplexlösung und rührte für den Verlauf einer Stunde im Rotationsverdampfer bei Raumtemperatur unter Normaldruck. Abschließend wurde die Suspension bei 90 - 100°C unter Vakuum (5 mbar) eingedampft. Das weiße Produkt wurde direkt zur Reduktion weiterverwendet.

In einem Labordrehrohrofen (Quarzglas, Durchmesser 5 cm, Länge in der Heizzone 20 cm) wurden 20 g des Pd-imprägnierten Produktes innerhalb von 90 Min bei einer Temperatur von 50°C mit einer Gasmischung aus 20 l/h Stickstoff und 1 l/h Wasserstoff bei einer Drehzahl des Ofens von 50 U/min reduziert.

Das fertige Produkt war von heller Farbe und Zeigte mittels transmissionselektronenmikroskopischer (TEM)-Analyse keine metallischen Palladium-Cluster mit Größen über 1,0 nm. Der Palladiumgehalt wurde naßchemisch zu 0,49 Gew.-% bestimmt. Mittels XPS fand man die drei vorn genannten Bindungsenergiezustände des Pd-3d_{5/2}-Photoelektrons (formell entsprechend den Oxidationsstufen +2, +1 und 0).

EXAFS-Messungen an dieser Probe zeigten ein Signal für Pd-O- oder Pd-N-Bindungsabstände von 2,02 ± 0,02 Å. Pd-Pd-Bindungsabstände von 2,74 ± 0,02 Å oder 3,04 ± 0,02 Å wurden nicht beobachtet.

### Beispiel 3

Mit dem Katalysator aus Beispiel 2 wurde in einer Druck-Apparatur unter Explosionsschutz die Umsetzung von Wasserstoff und Sauerstoff zu Wasserstoffperoxid in Suspensionsfahrweise bei 25 - 30°C untersucht.

Dazu wurden in den Druckreaktor 0,1 g Katalysator in 10 ml tert. Butanol als Lösungsmittel suspendiert und bei Raumtemperatur mit 0,1 l/min Wasserstoff für die Dauer von 30 min behandelt. Danach wurden auf den Reaktor 40 bar Stickstoff aufgepreßt und druckgeregelt 10 ml/min Wasserstoff sowie 100 ml/min Sauerstoff für die Dauer von 4,5 Stunden eindosiert. Aus der Gesamtmenge von 0,132 Mol Wasserstoff und 1,32 Mol Sauerstoff wurden nach Entspannen im Reaktionsaustrag 0,281 Gew.-% Wasserstoffperoxid mittels Jodometrie titrimetisch nachgewiesen.

Bei der Wiederholung des Versuches in deionisiertem Wasser als Lösungsmittel entstanden aus 0,129 Mol Wasserstoff und 1,29 Mol Sauerstoff insgesamt 0,196 Gew.-% Wasserstoffperoxid.

Wurde Methanol als Lösungsmittel eingesetzt, bildete sich aus 0,129 Mol Wasserstoff und 1,29 Mol Sauerstoff, 0,382 Gew.-% Wasserstoffperoxid im Reaktionsaustrag.

### Beispiel 4

Dieses Beispiel erläutert die einstufige Herstellung von Propylenoxid aus Propen, Wasserstoff und Sauerstoff an dem nach Beispiel 1 und 2 hergestellten Katalysator mit tert.-Butanol als Lösungsmittel.

In einem Glasdruckreaktor wurden in 60 ml tert.-Butanol als Lösungsmittel 1 g Katalysator aus Beispiel 2 unter Rühren suspendiert und für die Dauer von 30 Minuten mit 0,45 l/h Wasserstoff begast. Bei 45°C und einem Druck von 1 bar wurde sodann ein Gasgemisch aus 4 ml/h Propen, 0,45 l/h Wasserstoff, 0,45 l/h Sauerstoff und 1,5 l/h Stickstoff eingeleitet.

Aus gaschromatographischer Analyse fand man nach 5 Stunden einen Umsatz an Propen von 0,6 % mit einer Selektivität zu Propan von 9,4 % und zu Propylenoxid von 90,4 %.

### Beispiel 5

Dieses Beispiel erläutert die einstufige Herstellung von Propylenoxid aus Propen, Wasserstoff und Sauerstoff an dem nach Beispiel 1 und 2 hergestellten Katalysator mit Methanol als Lösungsmittel.

In einem Glasdruckreaktor wurden in 60 ml Methanol als Lösungsmittel 1 g Katalysator aus Beispiel 2 unter Rühren suspendiert und für die Dauer von 30 min mit Wasserstoff 0,45 l/h begast. Bei 22°C und einem Druck von 1 bar wurde sodann ein Gasgemisch aus 4 ml/l Propen, 0,9 l/h Wasserstoff, 0,9 l/h Sauerstoff und 3 l/h Stickstoff eingeleitet.

Aus gaschromatographischer Analyse fand man nach 17 Stunden einen Umsatz an Propen von 1,8 % mit einer Selektivität zu Propan von 5,2 % und zu Propylenoxid von 94,7 %.

### Beispiel 6

Dieses Beispiel erläutert die einstufige Herstellung von Propylenoxid aus Propen, Wasserstoff und Sauerstoff an dem nach Beispiel 1 und 2 hergestellten Katalysator mit Wasser als Lösungsmittel.

In einem Glasdruckreaktor wurden in 60 ml deionisiertem Wasser als Lösungsmittel 1 g Katalysator aus Beispiel 2 unter Rühren suspendiert und für die Dauer von 30 Min mit Wasserstoff 0,45 l/h begast. Bei 50°C und einem Druck von 1 bar wurde sodann ein Gasgemisch aus 4 ml/l Propen, 0,90 l/h Wasserstoff, 0,90 l/h Sauerstoff und 3 l/h Stickstoff eingeleitet.

Aus gaschromatographischer Analyse fand man nach 3 Stunden einen Umsatz an Propen von 1,4 % mit einer Selektivität zu Propan von 5,9 % und zu Propylenoxid von 94,0 %, nach 5 Stunden einen Umsatz an Propen von 1,8 % mit Selektivität zu Propylenoxid von 92,3 % und nach 20 Stunden einen Umsatz an Propen von 1,1 % mit Selektivität zum Propylenoxid von 91,1 %.

## Patentansprüche

1. Verfahren zur Herstellung von Epoxiden aus Olefinen, Wasserstoff und Sauerstoff durch heterogenkatalytische Umsetzung der Olefine unter Verwendung eines Oxidationskatalysators auf Basis von Titan- oder Vanadiumsilikaliten mit Zeolith-Struktur und einem Gehalt von 0,01 bis 20 Gew.-% an einem oder mehreren Platinmetallen aus der Gruppe Ruthenium, Rhodium, Palladium, Osmium, Iridium und Platin, wobei die Platinmetalle jeweils in mindestens zwei verschiedenen Bindungsenergiezuständen vorliegen, dadurch gekennzeichnet, daß die Platinmetalle durch Imprägnieren des Oxidationskatalysators mit Salzlösungen der Platinmetalle in der Oxidationsstufe +2 bis +4 und anschließende Hydrierung des getrockneten Katalysators in einer Wasserstoffatmosphäre bei Temperaturen von 20 bis 120°C derart aufgebracht sind, daß keine Metall-Metall-Bindungen wirksam werden, und daß die Titan- oder Vanadiumsilikalite eine Pentasil-Zeolith-Struktur mit röntgenographischer Zuordnung zur MFI-, MEL- oder MFI/MEL-Mischstruktur aufweisen.

2. Verfahren zur Herstellung von Epoxiden nach Anspruch 1 unter Verwendung eines Oxidationskatalysators mit einem Gehalt von 0,01 bis 20 Gew.-% Palladium, dadurch gekennzeichnet, daß das Palladium in zwei oder drei verschiedenen Bindungsenergiezuständen vorliegt.

3. Verfahren zur Herstellung von Epoxiden nach Anspruch 1 oder 2 unter Verwendung eines Oxidationskatalysators mit einem zusätzlichen Gehalt an einem oder mehreren Elementen aus der Gruppe Eisen, Kobalt, Nickel, Rhenium, Silber und Gold.

4. Verfahren zur Herstellung von Epoxiden nach den Ansprüchen 1 bis 3 unter Verwendung eines Oxidationskatalysators mit einem molaren Verhältnis von Titan und/oder Vanadium zur Summe aus Silicium plus Titan und/oder Vanadium von 0,01:1 bis 0,1:1.

5. Verfahren zur Herstellung von Epoxiden nach den Ansprüchen 1 bis 4, dadurch gekennzeichnet, daß man die Umsetzung in Gegenwart von Wasser durchführt.

6. Verfahren zur Herstellung von Propylenoxid nach den Ansprüchen 1 bis 5.

7. Verfahren zur Herstellung von Wasserstoffperoxid aus Wasserstoff und Sauerstoff, dadurch gekennzeichnet, daß man die Umsetzung heterogenkatalytisch unter Verwendung eines Oxidationskatalysators gemäß den Ansprüchen 1 bis 4 durchführt.

8. Oxidationskatalysator auf Basis von Titan- oder Vanadiumsilikaliten mit Zeolith-Struktur und einem Gehalt von 0,01 bis 20 Gew.-% an einem oder mehreren Platinmetallen aus der Gruppe Ruthenium, Rhodium, Palladium, Osmium, Iridium und Platin, wobei die Platinmetalle jeweils in mindestens zwei verschiedenen Bindungsenergiezuständen vorliegen und durch Imprägnieren des Oxidationskatalysators mit Salzlösungen der Platinmetalle in der Oxidationsstufe +2 bis +4 und anschließende Hydrierung des getrockneten Katalysators in einer Wasserstoffatmosphäre bei Temperaturen von 20 bis 120°C derart aufgebracht sind, daß keine Metall-Metall-Bindungen wirksam werden, dadurch gekennzeichnet, daß die Titan- oder Vanadiumsilikalite eine Pentasil-Zeolith-Struktur mit röntgenographischer Zuordnung zur MFI-, MEL- oder MFI/MEL-Mischstruktur aufweisen.

9. Oxidationskatalysator nach Anspruch 8 mit einem Gehalt von 0,01 bis 20 Gew.-% Palladium, wobei das Palladium in mindestens zwei verschiedenen Bindungsenergiezuständen vorliegt, dadurch gekennzeichnet, daß nahezu ausschließlich Palladium-Sauerstoff- oder Palladium-Stickstoff-Bindungsabstände von 2,02+0,02 Å und keine Palladium-Palladium-Abstände von 2,74+0,02 Å und 3,04+0,02 Å auftreten.

## Claims

1. A process for the preparation of epoxides from olefins, hydrogen and oxygen by reaction of the olefins under heterogeneous catalysis using an oxidation catalyst based on titanium or vanadium silicalites having a zeolite structure and containing from 0.01 to 20% by weight of one or more platinum metals selected from the group consisting of ruthenium, rhodium, palladium, osmium, iridium and platinum, the platinum metals each being present in at least two different bond energy states, wherein the platinum metals are applied by impregnation of the oxidation catalyst with salt solutions of the platinum metals in the oxidation state +2 to +4 and subsequent hydrogenation of the dried catalyst in a hydrogen atmosphere at from 20 to 120°C so that there are no metal-metal bonds and that the titanium or vanadium silicalites have a pentasil zeolite structure with assignment to the MFI, MEL or MFI/MEL mixed structure on the basis of X-ray analysis.

2. A process for the preparation of epoxides as claimed in claim 1 using an oxidation catalyst containing from 0.01 to 20% by weight of palladium, wherein the palladium is present in two or three different bond energy states.

3. A process for the preparation of epoxides as claimed in claim 1 or 2 using an oxidation catalyst additionally containing one or more elements selected from the group consisting of iron, cobalt, nickel, rhenium, silver and gold.

4. A process for the preparation of epoxides as claimed in any of claims 1 to 3 using an oxidation catalyst having a molar ratio of titanium and/or vanadium to the sum of silicon plus titanium and/or vanadium of from 0.01:1 to 0.1:1.

5. A process for the preparation of epoxides as claimed in any of claims 1 to 4, wherein the reaction is carried out in the presence of water.

6. A process for the preparation of propylene oxide as claimed in any of claims 1 to 5.

7. A process for the preparation of hydrogen peroxide from hydrogen and oxygen, wherein the reaction is carried out under heterogeneous catalysis using an oxidation catalyst as claimed in any of claims 1 to 4.

8. An oxidation catalyst based on titanium or vanadium silicalites having a zeolite structure and containing from 0.01 to 20% by weight of one or more platinum metals selected from the group consisting of ruthenium, rhodium, palladium, osmium, iridium and platinum, the platinum metals each being present in at least two different bond energy states and being applied by impregnation of the oxidation catalyst with salt solutions of the platinum metals in the oxidation state +2 to +4 and subsequent hydrogenation of the dried catalyst in a hydrogen atmosphere at from 20 to 120°C so that there are no metal-metal bonds, wherein the titanium or vanadium silicalites have a pentasil zeolite structure with assignment to the MFI, MEL or MFI/MEL mixed structure on the basis of X-ray analysis.

9. An oxidation catalyst as claimed in claim 8, containing from 0.01 to 20% by weight of palladium, the palladium being present in at least two different bond energy states, wherein virtually exclusively palladium-oxygen or palladium-nitrogen bond distances of 2.02 + 0.02 Å and no palladium-palladium distances of 2.74 + 0.02 Å and 3.04 + 0.02 Å occur.

## Revendications

1. Procédé de préparation d'époxydes à partir d'oléfines, d'hydrogène et d'oxygène, par réaction catalysée de façon hétérogène de l'oléfine en utilisant un catalyseur d'oxydation à base de silicalites de vanadium ou de titane à structure de zéolithe et ayant une teneur de 0,01-20% en poids en un ou plusieurs métaux du groupe du platine choisis dans le groupe formé par le ruthénium, le rhodium, le palladium, l'osmium, l'iridium et le platine, où les métaux du groupe du platine se trouvent à chaque fois dans au moins deux états d'énergie de liaison différents, caractérisé en ce que les métaux du groupe du platine sont déposés par imprégnation du catalyseur d'oxydation avec des solutions de sels des métaux du groupe du platine aux degrés d'oxydation +2 à +4 suivie par l'hydrogénation du catalyseur séché dans une atmosphère d'hydrogène à des températures de 20 à 120°C, de telle sorte qu'aucune liaison métal-métal ne devienne active, et que les silicalites de vanadium ou de titane présentent une structure de zéolithe pentasile dont l'étude aux rayons X montre une structure mixte MFI, MEL ou MFI/MEL.

2. Procédé de préparation d'époxydes selon la revendication 1, par utilisation d'un catalyseur d'oxydation ayant une teneur de 0,01-20% en poids de palladium, caractérisé en ce que le palladium se trouve dans deux ou trois états d'énergie de liaison différents.

3. Procédé de préparation d'époxydes selon la revendication 1 ou 2, par utilisation d'un catalyseur d'oxydation ayant une teneur supplémentaire en un ou plusieurs éléments choisis dans le groupe formé par le fer, le cobalt, le nickel, le rhénium, l'argent et l'or.

4. Procédé de préparation d'époxydes selon l'une quelconque des revendications 1 à 3, par utilisation d'un catalyseur d'oxydation ayant un rapport molaire du titane et/ou du vanadium à la somme du silicium, du titane et/ou du vanadium allant de 0,01:1 à 0,1:1.

5. Procédé de préparation d'époxydes selon l'une quelconque des revendications 1 à 4, caractérisé en ce que l'on mène la réaction en présence d'eau.

6. Procédé de préparation d'oxyde de propylène selon l'une quelconque des revendications 1 à 5.

7. Procédé de préparation de peroxyde d'hydrogène à partir d'hydrogène et d'oxygène, caractérisé en ce que l'on mène la réaction catalysée de façon hétérogène en utilisant un catalyseur d'oxydation selon l'une quelconque des revendications 1 à 4.

8. Catalyseur d'oxydation à base de silicalites de vanadium ou de titane à structure de zéolithe et ayant une teneur de 0,01-20% en poids en un ou plusieurs métaux du groupe du platine choisis dans le groupe formé par le ruthénium, le rhodium, le palladium, l'osmium, l'iridium et le platine, où les métaux du groupe du platine se trouvent à chaque fois dans au moins deux états d'énergie de liaison différents et sont déposés par imprégnation du catalyseur d'oxydation avec des solutions de sels des métaux du groupe du platine aux degrés d'oxydation +2 à +4 suivie par l'hydrogénation du catalyseur séché dans une atmosphère d'hydrogène à des températures de 20 à 120°C, de telle sorte qu'aucune liaison métal-métal ne devienne active, et que les silicalites de vanadium ou de titane présentent une structure de zéolithe pentasile dont l'étude aux rayons X montre une structure mixte MFI, MEL ou MFI/MEL.

9. Catalyseur d'oxydation selon la revendication 8, ayant une teneur de 0,01-20% en poids de palladium, où le palladium se trouve dans au moins deux états d'énergie de liaison différents, caractérisé en ce que l'on a presque exclusivement des distances de liaison palladium-oxygène ou palladium-azote de 2,02+0,02 Å et pas de distances de liaison palladium-palladium de 2,74+0,02 Å et de 3,04+0,02 Å.
